# EUROPÄISCHE PATENTSCHRIFT

(11) **EP 3 469 331 B1**
(45) Veröffentlichungstag und Bekanntmachung des Hinweises auf die Patenterteilung: **18.10.2023**
(21) Anmeldenummer: 17729105.1
(22) Anmeldetag: 08.06.2017
(51) Int. Cl.: G01N 1/20, G01N 1/14, G01N 33/14, G01N 1/10

(54) **PROBENENTNAHMEVORRICHTUNG ZUR ENTNAHME VON GETRÄNKEPROBEN AUS EINER GETRÄNKELEITUNG, DIE EIN UNTER DRUCK STEHENDES GASHALTIGES GETRÄNK ENTHÄLT**
SAMPLING DEVICE FOR TAKING BEVERAGE SAMPLES FROM A BEVERAGE LINE CONTAINING A GASEOUS BEVERAGE UNDER PRESSURE
DISPOSITIF D'ÉCHANTILLONNAGE PERMETTANT LE PRÉLÈVEMENT D'ÉCHANTILLONS DE BOISSON D'UNE CONDUITE DE BOISSON, LAQUELLE CONTIENT UNE BOISSON GAZEUSE PRESSURISÉE

(30) Priorität: 10.06.2016 DE 102016007094
(43) Veröffentlichungstag der Anmeldung: 17.04.2019
(73) Patentinhaber: QFood GmbH, Gundelfingen (DE)
(72) Erfinder: SEIDEL, Robert, 79102 Freiburg i.Br. (DE); KLAPPROTH, Holger, 79108 Freiburg I. Br. (DE); AUERSWALD, Martina, 61350 Bad Homburg (DE)
(74) Vertreter: Huwer, Andreas
(86) Internationale Anmeldenummer: PCT/EP2017/064011
(87) Internationale Veröffentlichungsnummer: WO 2017/211980

(56) Entgegenhaltungen:
- EP-A1- 0 367 899
- EP-A2- 1 035 407
- WO-A1-2004/034035
- DE-A1- 3 920 949
- DE-U1-202014 106 092
- US-A1- 2013 319 087

## Beschreibung

Die Erfindung betrifft eine Probenentnahmevorrichtung zur Entnahme von Getränkeproben aus einer Getränkeleitung, die ein unter Druck stehendes gashaltiges Getränk enthält.

Eine solche Probenentnahmevorrichtung, die einen Anschluss für eine Getränkeleitung aufweist, welche ein C0₂-haltiges Getränk enthält, ist aus DE 39 20 949 A1 bekannt. Probenentnahmevorrichtung weist eine Pumpe mit einem Pumpenzylinder auf, in dem ein beweglicher Kolben zwischen zwei Endlagen hin- und her bewegbar angeordnet ist. Der Kolben unterteilt den Pumpenzylinder in zwei gegeneinander abgedichtete Fluidräume. Der eine Fluidraum dient als Pumpenraum und ist über ein Wegeventil mit dem Anschluss für die Getränkeleitung verbindbar. Der andere Fluidraum ist mit einer Druckluftleitung verbunden, die über einen Druckregler an einer Druckluftquelle angeschlossen ist. Der Kolben steht einerseits unter dem Flüssigkeitsdruck eines mit dem Anschluss für die Getränkeleitung verbundenen Leitungssystems und andererseits unter dem Gasdruck der Druckluftleitung. Durch den Druck in der Getränkeleitung wird die Getränkeprobe durch das Leitungssystem hindurch in den Zylinder gedrückt, wobei sich der Kolben je nach eingestelltem Differenzdruck bis zum anderen Endanschlag bewegt, ohne dass die C0₂·haltige Getränkeprobe die Möglichkeit hat, zu entgasen oder zu schäumen. Danach schließt das Mehrwegeventil die Verbindung zwischen dem Pumpenraum und dem Anschluss für die Getränkeleitung und verbindet den Probenraum über eine Entleerungsleitung mit einer Abgabeöffnung, so dass die Getränkeprobe aus dem Zylinder mit Hilfe der Druckluft herausgedrückt werden kann. Das Leitungssystem hat außerdem eine Entlüftungsleitung mit einer Auslassöffnung.

Aus der WO 2004/034035 A1 ist eine Probenentnahmevorrichtung bekannt, die eine Pumpe aufweist, die eine Probenflüssigkeit aus einem Rohrabschnitt einer Verarbeitungsanlage in ein vertikales Rohrstück des Bypass-Systems saugt. Dann wird ein erstes Ventil geschlossen, um mit Hilfe der Pumpe ein Vakuum in der Probenflüssigkeit zu erzeugen. Nachdem ein zweites Ventil wird geschlossen wurde, wird die Probenflüssigkeit mit Hilfe eines Kolbens in dem Rohrstück komprimiert. Danach werden die Eigenschaften der Probenflüssigkeit mittels einer Messvorrichtung gemessen. Die Probe kann dann wieder an die Verarbeitungsanlage zurückgegeben werden. Durch das Anlegen des Vakuums an die Probenflüssigkeit und das anschließende Komprimieren der Probenflüssigkeit werden eventuelle, in der Probenflüssigkeit enthaltene Gasblasen aus der Probenflüssigkeit entfernt.

Aus der DE 20 2014 106 092 U1 ist eine Milchsammelvorrichtung mit einem Luftabscheider bekannt. Zur Einsaugung der Milch in den Luftabscheider ist dieser über eine Vakuumpumpe mit Unterdruck beaufschlagbar. Während ihrer Verweilzeit im Luftabscheidebehälter hat die Milch Gelegenheit zu entgasen bzw. sich von Luftbestandteilen zu trennen.

Auch aus der EP 1 035 407 A2 ist eine Milchsammelvorrichtung mit einem Luftabscheider bekannt. Dieser hat einen unter Unterdruck stehenden Behälter, der mit einer Förderleitung für unter Einschluss von Luft und Schaum geförderte Milch.

Aus der EP 0 367 899 A1 ist Anordnung bekannt, bei der eine Durchflussleitung an einen Luftabscheider angeschlossenen, dessen Kopfraum mit einer Unterdruckquelle in Verbindung steht.

Aus DE 103 52 924 A1 ist ferner eine Probenentnahmevorrichtung bekannt, die einen Anschluss für eine Getränkeleitung einer Getränke-Produktionsanlage hat. Durch die Getränkeleitung fließt ein unter Druck stehendes gashaltiges Getränk, wie zum Beispiel Bier. Der Anschluss für die Getränkeleitung ist über eine Zuleitung mit einer Abgabeöffnung für die Getränkeprobe verbunden, die an einer Einlassöffnung einer Durchflussmesszelle eines Infrarot-Spektrometers angeschlossen ist. Zwischen dem Anschluss für die Getränkeleitung und der Abgabeöffnung ist ein Grobpartikelfilter in der Zuleitung angeordnet, der eine Porengröße zwischen 1 µm und 0,1 mm aufweist. Die Auslassöffnung der Durchflussmesszelle ist über eine Fluidleitung, in der ein Leitfähigkeitssensor, ein pH-Sensor, ein Trübungssensor, ein Nadelventil, ein Absperrventil und eine Ansaugpumpe angeordnet sind, mit einem Auslass für die Getränkeprobe verbunden. Zwischen dem Trübungssensor und dem Nadelventil zweigt eine Rücklaufleitung von der Fluidleitung ab. Über die Rücklaufleitung kann die Getränkeprobe bei Bedarf in den Produktionsprozess zurückgeführt werden. Dies entspricht jedoch nicht den bei der Herstellung von Lebensmitteln anzuwendenden Sicherheitsauflagen gemäß den Leitlinien des European Hygienic Engineering & Design Group (EHEDG), wonach keine Flüssigkeiten aus der Probenentnahmevorrichtung zurück in die Getränkeleitung der Lebensmittelabfülleinrichtung gelangen dürfen. Das in Strömungsrichtung hinter der Durchflussmesszelle angeordnete Nadelventil und das in Strömungsrichtung hinter diesem angeordnet Absperrventil dienen dazu, in der Durchflussmesszelle bei der Analyse von kohlensäurehaltigen Getränken einen Druck von 2 bis 5 Atmosphären aufrecht zu erhalten. Druckempfindliche Analysegeräte, wie z.B. das QFOOD QUANTOS^{®} sind jedoch für so hohe Drücke nicht geeignet. Mit der Probenentnahmevorrichtung kann zwar auch unter normalen Druckbedingungen gearbeitet werden, wenn das Nadelventil und/oder das Absperrventil geöffnet werden. Dies hat aber den Nachteil, dass sich dann Gasblasen in der Getränkeprobe bilden können, die Funktionsstörungen des Analysegeräts verursachen können, insbesondere wenn die Getränkeprobe durch sehr dünne Fluidkanäle und/oder eine Durchflussmesszelle transportiert wird, in der die Getränkeprobe zwischen in geringem Abstand parallel zueinander angeordneten Wänden eine dünne Schicht bildet.

Aus der US 2013/0319087 A1 ist eine Chromatographievorrichtung bekannt, die eine Probenentnahmevorrichtung zur Entnahme von Proben aus einer Leitung aufweist, die mit einem Vorratsbehälter für einen Eluent verbunden ist, wobei die Probenentnahmevorrichtung
- einen Anschluss für die Leitung,
- eine Pumpe mit einem Pumpenraum hat, der durch Wandungen begrenzt ist, die zum Expandieren und Komprimieren des Pumpraums mittels des Antriebs aufeinander zu- und voneinander wegbewegbar sind,
- eine Abgabeöffnung für die Probe,
- ein verstellbare Ventile aufweisendes Leitungssystem, welches mit dem Anschluss für die Leitung, dem Pumpenraum der Abgabeöffnung und einer Auslassöffnung für Abfall verbunden ist, und
- eine Steuereinrichtung hat,
wobei das Leitungssystem derart ausgestaltet ist und die Steuereinrichtung derart mit der Pumpe und den Ventilen in Steuerverbindung steht,
i) dass in einer ersten Ventilkonfiguration, bei welcher der Pumpenraum mit dem Anschluss für die Leitung verbunden und von der Auslassöffnung sowie der Abgabeöffnung getrennt ist, eine Probe, deren Volumen kleiner ist als das maximale Volumen des Pumpenraums, vom Anschluss für die Leitung in den Pumpenraum einbringbar ist,
ii) dass danach eine zweite Ventilkonfiguration eingestellt wird, in welcher der Pumpenraum vom Anschluss für die Leitung, von der Auslassöffnung sowie der Abgabeöffnung getrennt ist, und der Pumpenraum derart expandiert wird, dass zum Entgasen der Probe ein Unterdruck im Pumpenraum entsteht,
iii) dass danach eine dritte Ventilkonfiguration eingestellt wird, in welcher der Pumpenraum mit der Auslassöffnung verbunden und von dem Anschluss für die Leitung sowie der Abgabeöffnung getrennt ist, und der Pumpenraum derart komprimiert wird, dass ein in einem oberen Teil des Pumpenraums befindliches erstes Teilvolumen zu der Auslassöffnung hin verdrängt und ein in einem unteren Teil des Pumpenraums befindliches zweites Teilvolumen im Pumpenraum verbleibt,
iv) dass danach eine vierte Ventilkonfiguration eingestellt, in welcher der Pumpenraum über den Partikelfilter mit der Abgabeöffnung verbunden und von dem Anschluss für die Leitung, der Zuleitung für das Rückspülmedium sowie der Auslassöffnung getrennt ist, und der Pumpenraum zum Verdrängen der darin befindlichen Probe zur Abgabeöffnung hin weiter komprimiert wird.

Die vorbekannte Chromatographievorrichtung ist zum Untersuchen von Blutproben vorgesehen und weist eine mit der Abgabeöffnung verbundene Trennsäule auf, an der ein in der Blutprobe enthaltener Analyt adsorbiert wird. Dieser wird mittels des Eluents aus der Trennsäule herausgelöst und danach chromatographisch untersucht.

Es besteht deshalb die Aufgabe, eine robust aufgebaute Probenentnahmevorrichtung zu schaffen, mittels der auf einfache Weise aus einer Getränkeleitung, die ein unter Druck stehendes gashaltiges Getränk enthält, eine Getränkeprobe entnehmbar und unter Normaldruck an einer Abgabeöffnung einem Infrarotspektrometer zuführbar ist. Dabei sollen an der Abgabeöffnung Gasblasen in der Getränkeprobe vermieden werden. Die Probenentnahmevorrichtung soll außerdem einen wartungsarmen und störungsfreien Betrieb ermöglichen.

Erfindungsgemäß wird diese Aufgabe mit den Merkmalen des Anspruchs 1 gelöst.

In Vorteilhafter Weise erfüllt der Pumpenraum dabei eine Doppelfunktion, bei welcher er einerseits zum Entgasen der Getränkeprobe dient und andererseits für den Transport der entgasten Getränkeprobe vom Pumpenraum zu der Abgabeöffnung für die Getränkeprobe benutzt wird. Dies ermöglicht einen einfachen und robusten Aufbau der Probenentnahmevorrichtung. Da die Getränkeprobe an der Abgabeöffnung im Wesentlichen unter Atmosphärendruck abgegeben wird, kann die Probenentnahmevorrichtung auch mit Analysegeräten kombiniert werden, bei denen der Analyt keinen oder nur einen sehr geringen Überdruck aufweisen darf. Weil die Getränkeprobe entgast wird, bevor sie zu der Abgabeöffnung gelangt, und das aus der Getränkeprobe ausgetretene, im oberen Teil des Pumpenraums befindliche Gas über die Auslassöffnung abgeführt wird, werden an der Abgabeöffnung Gasblasen in der Getränkeprobe, die zu Funktionsstörungen des Analysegeräts führen können, vermieden. Die Pumpe ist bevorzugt als Spritzenpumpe oder als Kolbenpumpe ausgestaltet. Auch durch diese Maßnahme wird einsss einfacher und robuster Aufbau der Probenentnahmevorrichtung ermöglicht.

Bei der Erfindung weist die Probenentnahmevorrichtung zum Filtern der Getränkeprobe einen Partikelfilter mit einem Einlass und einem Auslass für die Getränkeprobe auf, wobei das Leitungssystem mit dem Einlass des Partikelfilters, dem Auslass des Partikelfilters und einer Zuleitung für ein Rückspülmedium verbunden ist, dass in der vierten Ventilkonfiguration der Pumpenraum über den Partikelfilter mit der Abgabeöffnung verbunden ist, dass in der ersten, zweiten, dritten und vierten Ventilkonfiguration der Pumpenraum von der Zuleitung für das Rückspülmedium getrennt ist, dass in einer fünften Ventilkonfiguration die Zuleitung für das Rückspülmedium über den Partikelfilter mit der Auslassöffnung verbunden ist, und dass die Steuereinrichtung derart mit den Ventilen in Steuerverbindung steht, dass nach Schritt iv) aus Anspruch 1 zum Rückspülen des Partikelfilters die fünfte Ventilkonfiguration eingestellt wird. Durch den Partikelfilter wird verhindert, dass eventuelle, in der Getränkeprobe enthaltene Partikel zur Abgabeöffnung für die Probe und damit in ein daran angeschlossenes, zum Untersuchen der Getränkeprobe vorgesehenes Analysegerät gelangen können. Da der Partikelfilter nach Gebrauch automatisch rückgespült wird, können Partikel, die vom Partikelfilter aus der Getränkeprobe zurückgehalten werden, auf einfache Weise wieder aus dem Partikelfilter entfernt und über die Auslassöffnung aus der Probenentnahmevorrichtung abgeführt werden. Dies ermöglicht einen wartungsarmen und störungsfreien Betrieb der Probenentnahmevorrichtung.

Bei einer bevorzugten Ausgestaltung der Erfindung ist die Steuereinrichtung derart ausgestaltet, dass die Schritte ii) und iii) aus Anspruch 1 mindestens einmal wiederholt werden, bevor Schritt iv) aus Anspruch 1 durchgeführt wird. Dadurch kann der Gasgehalt der Getränkeprobe weiter reduziert werden.

Bevorzugt ist in der zweiten und/oder dritten Ventilkonfiguration der Pumpenraum vom Einlass des Partikelfilters getrennt. Dadurch wird verhindert, dass Gasblasen aus dem Pumpenraum in den Partikelfilter gelangen können. Auch in der ersten Ventilkonfiguration kann der Pumpenraum vom Einlass des Partikelfilters getrennt sein.

Bei einer vorteilhaften Ausführungsform der Erfindung weist der Partikelfilter einen Vorfilter und einen damit in Reihe geschalteten Feinfilter auf, wobei der Einlass des Partikelfilters am Vorfilter und der Auslass des Partikelfilters am Feinfilter angeordnet ist. Durch den Feinfilter können Partikel bis zu einer Größe von vorzugsweise 0,4 µm aus der Getränkeprobe herausgefiltert werden. Der dem Feinfilter vorgeschaltete Vorfilter filtert größere Partikel aus der Getränkeprobe heraus. Dadurch wird die Standzeit des Feinfilters vergrößert.

Bei einer bevorzugten Ausgestaltung der Erfindung ist der Anschluss für die Getränkeleitung über eine erste Leitung mit dem Pumpenraum verbunden ist, wobei in der ersten Leitung ein erstes Absperrventil angeordnet ist, wobei der Pumpenraum über eine zweite Leitung, die in den Pumpenraum mündet oder an einer zwischen diesem und dem ersten Absperrventil angeordneten ersten Verzweigungsstelle an der ersten Leitung angeschlossen ist, mit einem ersten Fluidanschluss eines ersten Dreiwegeventils verbunden ist, und wobei ein zweiter Fluidanschluss des ersten Dreiwegeventils mit der Auslassöffnung und ein dritter Fluidanschluss des ersten Dreiwegeventils mit dem Einlass des Partikelfilters verbunden ist. Dabei ermöglicht das Dreiwegeventil einen einfachen Aufbau des Leitungssystems.

Bei einer anderen zweckmäßigen Ausführungsform der Erfindung ist der Anschluss für die Getränkeleitung über eine erste Leitung mit dem Pumpenraum verbunden ist, wobei in der ersten Leitung ein erstes Absperrventil angeordnet ist, wobei der Pumpenraum über eine zweite Leitung, die in den Pumpenraum mündet oder an einer zwischen diesem und dem ersten Absperrventil angeordneten ersten Verzweigungsstelle an der ersten Leitung angeschlossen ist, mit dem Einlass des Partikelfilters verbunden ist, wobei in der zweiten Leitung ein zweites Absperrventil angeordnet ist, und wobei am Pumpenraum und/oder an der ersten Leitung zwischen dem Pumpenraum und dem ersten Absperrventil und/oder an der zweiten Leitung zwischen dem Pumpenraum und dem zweiten Absperrventil eine Anschlussstelle vorgesehen ist, die über ein drittes Absperrventil mit der Auslassöffnung für den Abfall verbunden ist. Anstelle des ersten Dreiwegeventils können also auch zwei Absperrventile vorgesehen sein.

Die erfindungsgemäße Probenentnahmevorrichtung ist bevorzugt kompatibel zu den Leitlinien des European Hygienic Engineering & Design Group (EHEDG) ausgestaltet. Insbesondere kann die Probenentnahmevorrichtung derart ausgestaltet sein, dass keine Flüssigkeiten, die hinter dem ersten Absperrventil in der Probenentnahmevorrichtung angeordnet sind, in die Getränkeleitung zurück fließen können. Das kann dadurch erreicht werden, dass das erste Absperrventil beim Komprimieren des Pumpenraums stets geschlossen ist.

Bei einer Weiterbildung der Erfindung ist in dem Leitungssystem zwischen dem Anschluss für die Getränkeleitung und dem Einlass des Partikelfilters und/oder zwischen dem Pumpenraum und dem Einlass des Partikelfilters mindestens ein mit der Steuereinrichtung verbundener Sensor zum Detektieren des Getränks angeordnet, wobei die Steuereinrichtung derart ausgestaltet ist, dass der Einlass des Partikelfilters mittels des ersten Dreiwegeventils oder des zweiten Absperrventils abgesperrt ist, wenn der Sensor das Getränk nicht detektiert. Der Einlass des Partikelfilters ist dann gesperrt ist, wenn der Sensor mit einem Gas und/oder mit einer Reinigungs-, Desinfektions- und/oder Spülflüssigkeit in Kontakt gerät, die im Rahmen eines Cleaning in Place Reinigungsverfahren (CIP) durch die Getränkeleitung geleitet wird. Mit Hilfe des in der Nahrungsmittelindustrie üblichen CIP Reinigungsverfahrens kann eine Produktionsanlage für Getränke ohne wesentliche Demontage auf den Flächen, die mit dem Getränk in Berührung gelangen, gereinigt werden. Die erfindungsgemäße Probenentnahmevorrichtung ist also auch für eine Verwendung in Produktionsanlagen geeignet, die mit dem CIP Reinigungsverfahren gereinigt bzw. desinfiziert werden.

Zweckmäßigerweise weist der Sensor einen Leitfähigkeitssensor und/oder einen pH-Wertsensor auf. Dies ermöglicht eine kostengünstige Ausgestaltung des Sensors.

Bei einer vorteilhaften Ausführungsform der Erfindung ist der Auslass des Partikelfilters mit einem ersten Fluidanschluss eines zweiten Dreiwegeventils verbunden, wobei ein zweiter Fluidanschluss des zweiten Dreiwegeventils mit der Abgabeöffnung für die Getränkeprobe und ein dritter Fluidanschluss des zweiten Dreiwegeventils mit der Zuleitung für das Rückspülmedium verbunden ist. Dabei ermöglicht das zweite Dreiwegeventil einen einfachen Aufbau des Leitungssystems.

Bei einer anderen Ausgestaltung der Erfindung ist vorgesehen, dass der Auslass des Partikelfilters über eine dritte Leitung, in der ein viertes Absperrventil angeordnet ist, mit der Abgabeöffnung für die Getränkeprobe verbunden ist, und dass zwischen dem Auslass des Partikelfilters und dem vierten Absperrventil eine zweite Verzweigungsstelle angeordnet ist, an der die dritte Leitung über ein fünftes Absperrventil mit der Zuleitung für das Rückspülmedium verbunden ist. Anstelle des zweiten Dreiwegeventils können also auch zwei Absperrventile vorgesehen sein.

Bei einer bevorzugten Ausgestaltung der Erfindung ist vorgesehen, dass der Anschluss für die Getränkeleitung über eine erste Leitung mit dem Pumpenraum verbunden ist, dass in der ersten Leitung das erste Absperrventil und ein sechstes Absperrventil in Reihe geschaltet sind, dass der Pumpenraum oder eine zwischen dem sechsten Absperrventil und dem Pumpenraum in der ersten Leitung vorgesehene erste Verzweigungsstelle über die zweite Leitung mit dem Einlass des Partikelfilters verbunden ist, und dass zwischen dem ersten Absperrventil und dem sechsten Absperrventil eine dritte Verzweigungsstelle angeordnet ist, an der die erste Leitung über eine vierte Leitung, in der ein siebtes Absperrventil angeordnet ist, mit der Auslassöffnung oder einer weiteren Auslassöffnung für Abfall verbunden ist.

Dabei können das erste, sechste und siebte Absperrventil beim Durchleiten einer Reinigungs-, Desinfektions- und/oder Spülflüssigkeit durch die Getränkeleitung mittels der Steuereinrichtung derart konfiguriert werden, dass das erste und siebte Absperrventil geöffnet und das sechste Absperrventil geschlossen ist. Die Reinigungs-, Desinfektions- und/oder Spülflüssigkeit kann dann vom Anschluss für die Getränkeleitung über das erste Absperrventil und die erste Leitung zur dritten Verzweigungsstelle und von dort über die vierte Leitung und das siebte Absperrventil zur Auslassöffnung fließen. Da das sechste Absperrventil in dieser Konfiguration gesperrt ist, kann die Reinigungs-, Desinfektions- und/oder Spülflüssigkeit weder in den Pumpenraum noch in den Partikelfilter gelangen. In einer weiteren Konfiguration, die zum Befüllen des Pumpenraums mit der Getränkeprobe dient, sind das erste und sechste Absperrventil geöffnet und das siebte Absperrventil ist geschlossen. Bei Bedarf können das erste, sechste und siebte Absperrventil über die Steuereinrichtung auch so eingestellt werden, dass das siebte Absperrventil geöffnet und das erste und sechste Absperrventil geschlossen sind. Dadurch kann auch dann keine Flüssigkeit aus dem Pumpenraum in die Getränkeleitung zurückfließen, wenn das erste und/oder sechste Absperrventil einmal undicht werden sollten.

Nachfolgend sind Ausführungsbeispiele der Erfindung anhand der Zeichnung näher erläutert. Es zeigt
- Fig. 1: eine schematische Darstellung eines ersten Ausführungsbeispiels der Probenentnahmevorrichtung,
- Fig. 2: eine schematische Darstellung eines zweiten Ausführungsbeispiels der Probenentnahmevorrichtung,
- Fig. 3: eine schematische Darstellung eines dritten Ausführungsbeispiels der Probenentnahmevorrichtung,
- Fig. 4: eine schematische Darstellung eines vierten Ausführungsbeispiels der Probenentnahmevorrichtung,
- Fig. 5: eine schematische Darstellung eines fünften Ausführungsbeispiels der Probenentnahmevorrichtung, und
- Fig. 6: eine schematische Darstellung eines sechsten Ausführungsbeispiels der Probenentnahmevorrichtung.

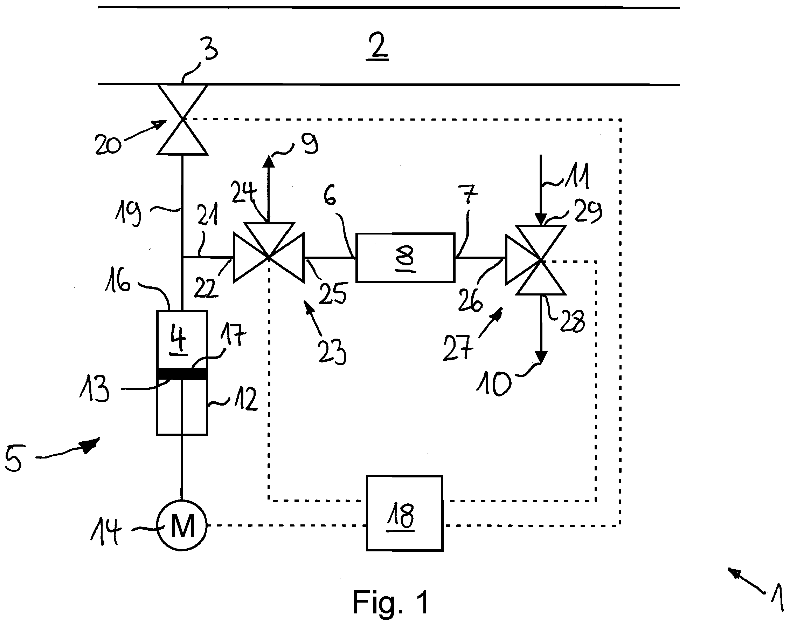

Eine in Fig. 1 im Ganzen mit 1 bezeichnete Probenentnahmevorrichtung dient zur Entnahme von Getränkeproben aus einer Getränkeleitung 2 in einer Getränkeproduktionsanlage. In der Getränkeleitung 2 ist ein unter Druck stehendes gashaltiges Getränk angeordnet, beispielsweise Bier oder eine kohlensäurehaltige Limonade.

Die Probenentnahmevorrichtung 1 hat einen in Fig. 1 nur schematisch dargestellten Anschluss 3, der fest mit der Getränkeleitung 2 verbunden ist. Der Anschluss 3 ist über ein Leitungssystem das mittels in der Zeichnung nicht näher dargestellten Aktoren verstellbare Ventile aufweist, mit einem Pumpenraum 4 einer Pumpe 5, einem einen Einlass 6 und einen Auslass 7 aufweisenden Partikelfilter 8 zum Filtern der Getränkeprobe, einer Auslassöffnung 9 für Abfall, einer Abgabeöffnung 10 für die Getränkeprobe und einer Zuleitung 11 für ein Rückspülmedium verbunden. An der Abgabeöffnung 10 kann ein in der Zeichnung nicht näher dargestelltes Analysegerät, das zur Untersuchung der Getränkeprobe dient, angeschlossen sein, wie z.B. das QFOOD QUANTOS^{®}.

Die Pumpe 5 ist als Spritzenpumpe ausgestaltet, die einen in einem Zylinder 12 axial verschiebbaren Kolben 13 aufweist, der mittels eines in der Zeichnung nur schematisch dargestellten Antriebs 14 verstellbar ist. Der Zylinder 12 und der Kolben 13 begrenzen einen Pumpenraum 4, der an seinem oberen Ende durch eine obere Wandung 16, an seinem unteren Ende durch eine durch den Kolben 13 gebildete untere Wandung 17 und seitlich durch die Innenwand des Zylinders 12 begrenzt wird, welche den Pumpenraum 4 umgrenzt. Zum Expandieren und Komprimieren des Pumpraums 4 ist der Kolben 13 mittels des Antriebs 14 auf die obere Wandung 16 zu- und von dieser wegbewegbar.

Mit Hilfe der Ventile ist das Leitungssystem wie folgt konfigurierbar:
I) In einer ersten Ventilkonfiguration ist der Pumpenraum 4 mit dem Anschluss 3 für die Getränkeleitung 2 verbunden und von der Auslassöffnung 9 für den Abfall, der Abgabeöffnung 10 für die Getränkeprobe sowie der Zuleitung 11 für das Rückspülmedium getrennt.
II) In einer zweiten Ventilkonfiguration ist der Pumpenraum 4 vom Anschluss 3 für die Getränkeleitung, von der Auslassöffnung 9 für den Abfall, der Abgabeöffnung 10 für die Getränkeprobe sowie der Zuleitung 11 für das Rückspülmedium getrennt.
III) In einer dritten Ventilkonfiguration ist der Pumpenraum 4 mit der Auslassöffnung 9 für den Abfall verbunden und von dem Anschluss 3 für die Getränkeleitung 2, der Abgabeöffnung 10 für die Getränkeprobe sowie der Zuleitung 11 für das Rückspülmedium getrennt.
IV) In einer vierten Ventilkonfiguration ist der Pumpenraum 4 mit einem Einlass 6 des Partikelfilters 8 und der Auslass 7 des Partikelfilters 8 mit der Abgabeöffnung 10 für die Getränkeprobe verbunden. Außerdem ist der Pumpenraum 4 von dem Anschluss 3 für die Getränkeleitung 2, der Auslassöffnung 9 für den Abfall sowie der Zuleitung 11 für das Rückspülmedium getrennt.
V) In einer fünften Ventilkonfiguration ist die Zuleitung 11 für das Rückspülmedium über den Partikelfilter 8 mit der Auslassöffnung 9 für den Abfall verbunden und vom Anschluss 3 für die Getränkeleitung 2, dem Pumpenraum 4 und der Abgabeöffnung 10 getrennt.

Bei dem in Fig. 1 gezeigten ersten Ausführungsbeispiel ist der Anschluss 3 für die Getränkeleitung 2 über eine erste Leitung 19 mit dem oberen Ende des Pumpenraums 4 verbunden. Deutlich ist erkennbar, dass die erste Leitung 19 in an der oberen Wandung 16 in den Pumpenraum 4 mündet.

In der ersten Leitung ist dicht benachbart zum Anschluss 3 ein erstes Absperrventil 20 angeordnet, mittels dem die erste Leitung 19 zur Getränkeleitung 2 hin abgesperrt werden kann. Zwischen dem ersten Absperrventil 20 und dem Pumpenraum 4 hat die erste Leitung 19 eine erste Verzweigungsstelle, an der die erste Leitung 19 über eine zweite Leitung 21 mit einem ersten Fluidanschluss 22 eines ersten Dreiwegeventils 23 verbunden ist. Ein zweiter Fluidanschluss 24 des ersten Dreiwegeventils 23 mit der Auslassöffnung 9 für den Abfall und ein dritter Fluidanschluss 25 des ersten Dreiwegeventils 23 ist mit dem Einlass 6 des Partikelfilters 8 verbunden.

Der Auslass 7 Partikelfilters 8 ist mit einem ersten Fluidanschluss 26 eines zweiten Dreiwegeventils 27 verbunden, ein zweiter Fluidanschluss 28 des zweiten Dreiwegeventils 27 ist mit der Abgabeöffnung 10 für die Getränkeprobe und ein dritter Anschluss 29 des zweiten Dreiwegeventils 27 ist mit der Zuleitung 11 für das Rückspülmedium verbunden.

Wie in Fig. 1 erkennbar ist, weist die Probenentnahmevorrichtung 1 außerdem eine Steuereinrichtung 18 auf. Diese steht derart mit dem ersten Absperrventil 20, den Dreiwegeventilen 23, 27 und dem Antrieb 14 der Pumpe 5 in Steuerverbindung, dass folgende Schritte durchlaufen werden:
i) In der ersten Ventilkonfiguration wird eine Getränkeprobe, deren Volumen kleiner ist als das maximale Volumen des Pumpenraums 4, vom Anschluss 3 für die Getränkeleitung 2 in den Pumpenraum 4 eingebracht. Das kann beispielsweise dadurch erreicht werden, dass die erste Ventilkonfiguration für eine an das Volumen der Getränkeprobe und den Volumenstrom in der ersten Leitung 19 angepasste, vorbestimmte Zeitdauer eingestellt wird.
ii) Danach wird eine zweite Ventilkonfiguration eingestellt und der Pumpenraum 4 wird derart expandiert wird, dass Gas aus der Getränkeprobe austritt. Dabei wird die Dauer, während welcher der Pumpenraum 4 expandiert ist, derart gewählt, dass Gas in nennenswerter Menge aus der Getränkeprobe austreten und in den oberhalb der Getränkeprobe befindlichen Teil des Pumpenraums 4 gelangen kann.
iii) Danach wird die dritte Ventilkonfiguration eingestellt und der Pumpenraum 4 derart komprimiert wird, dass aus der Getränkeprobe ausgetretenes, im oberen Teil des Pumpenraums 4 befindliches Gas zur Auslassöffnung 9 für den Abfall hin verdrängt wird und die im unteren Teil des Pumpenraums 4 befindliche Getränkeprobe im Wesentlichen im Pumpenraum 4 verbleibt.
iv) Die Schritte ii) und iii) aus Anspruch 1 werden mindestens einmal wiederholt.
v) Danach wird die vierte Ventilkonfiguration eingestellt und der Pumpenraum 4 wird derart weiter komprimiert, dass die darin befindliche Getränkeprobe zur Abgabeöffnung 10 hin verdrängt wird. Dabei durchströmt die Getränkeprobe den Partikelfilter 8.
vi) Danach wird zum Rückspülen des Partikelfilters 8 die fünfte Ventilkonfiguration eingestellt. Dabei fließt sauberes Wasser aus der Zuleitung 11 entgegen der Strömungsrichtung der Getränkeprobe durch den Partikelfilters 8 hindurch zur Auslassöffnung 9, wo es beispielsweise in die Kanalisation oder einen Auffangbehälter eingeleitet wird.

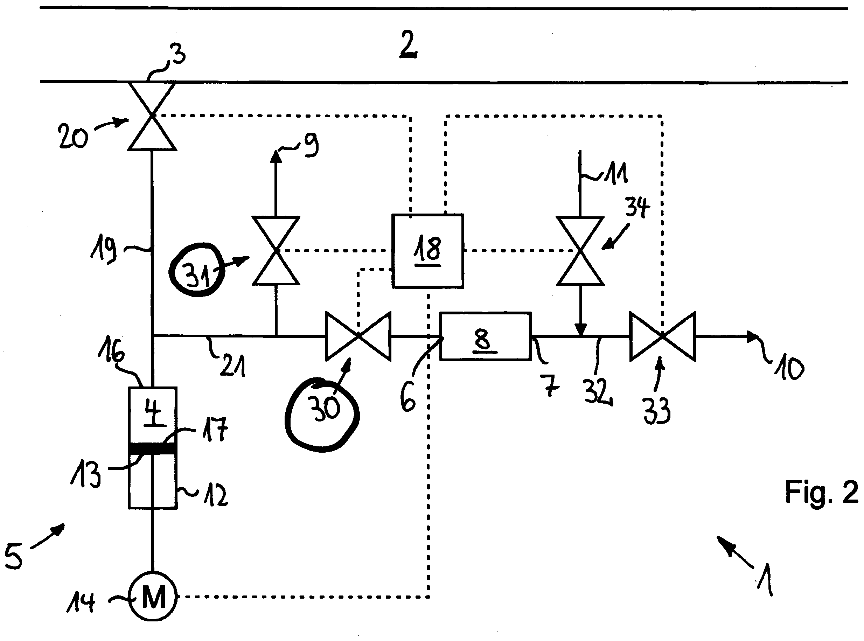

Bei dem in Fig. 2 abgebildeten zweiten Ausführungsbeispiel weist die Probenentnahmevorrichtung 1 anstelle des ersten Dreiwegeventils ein zweites und ein drittes Absperrventil 30, 31 auf. Das zweite Absperrventil 30 ist zwischen der ersten Leitung 19 und dem Partikelfilter 8 in der zweiten Leitung 21 angeordnet. Die zweite Leitung 21 weist zwischen der ersten Leitung 19 und dem zweiten Absperrventil 30 eine Anschlussstelle auf, die über das dritte Absperrventil 31 mit der Auslassöffnung 9 für den Abfall verbunden ist.

Der Auslass 7 des Partikelfilters 8 ist über eine dritte Leitung 32, in der ein viertes Absperrventil 33 angeordnet ist, mit der Abgabeöffnung 10 für die Getränkeprobe verbunden. Zwischen dem Auslass 7 des Partikelfilters 8 und dem vierten Absperrventil 33 ist eine zweite Verzweigungsstelle angeordnet, an der die dritte Leitung 32 über ein fünftes Absperrventil 34 mit der Zuleitung 11 für das Rückspülmedium verbunden ist.

Die Steuereinrichtung 18 steht derart mit den Absperrventilen 20, 30, 33, 34 und dem Antrieb 14 der Pumpe 5 in Steuerverbindung, dass die oben genannten Schritte i) bis vi) durchlaufen werden. Im Übrigen entspricht das zweite Ausführungsbeispiel im Wesentlichen dem Ausführungsbeispiel aus Fig. 1.

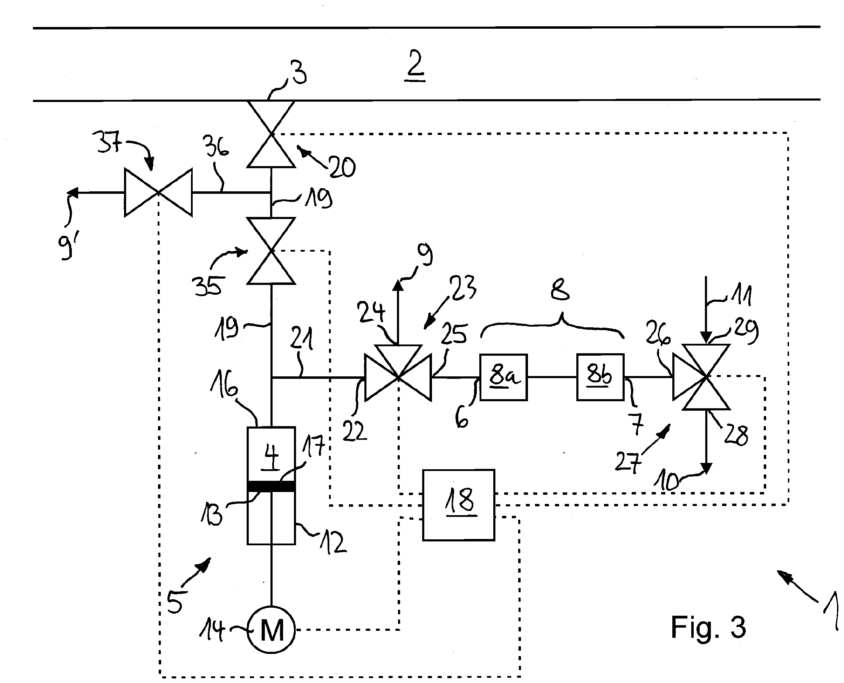

Das in Fig. 3 abgebildete dritte Ausführungsbeispiel entspricht im Wesentlichen dem ersten Ausführungsbeispiel, weist jedoch zusätzlich ein sechstes und ein siebtes Absperrventil 35, 37 auf. Das sechste Absperrventil 35 ist in der ersten Leitung 19 zwischen dem ersten Absperrventil 20 und der ersten Verzweigungsstelle angeordnet, an der die zweite Leitung 21 von der ersten Leitung 19 abzweigt. Zwischen dem ersten Absperrventil 20 und dem sechsten Absperrventil 35 ist eine dritte Verzweigungsstelle angeordnet, an der die erste Leitung 19 über eine vierte Leitung 36, in der ein siebtes Absperrventil 37 angeordnet ist, mit einer weiteren Auslassöffnung 9' für Abfall verbunden ist. Das erste, sechste und siebte Absperrventil 20, 35, 37 sind beim Durchleiten einer Reinigungs-, Desinfektions- und/oder Spülflüssigkeit durch die Getränkeleitung 2 derart konfiguriert, dass das erste und siebte Absperrventil 20, 37 geöffnet und das sechste Absperrventil 35 geschlossen ist. Zum Befüllen des Pumpenraums 4 mit der Getränkeprobe sind das erste und sechste Absperrventil 20, 35 geöffnet und das siebte Absperrventil 37 ist geschlossen.

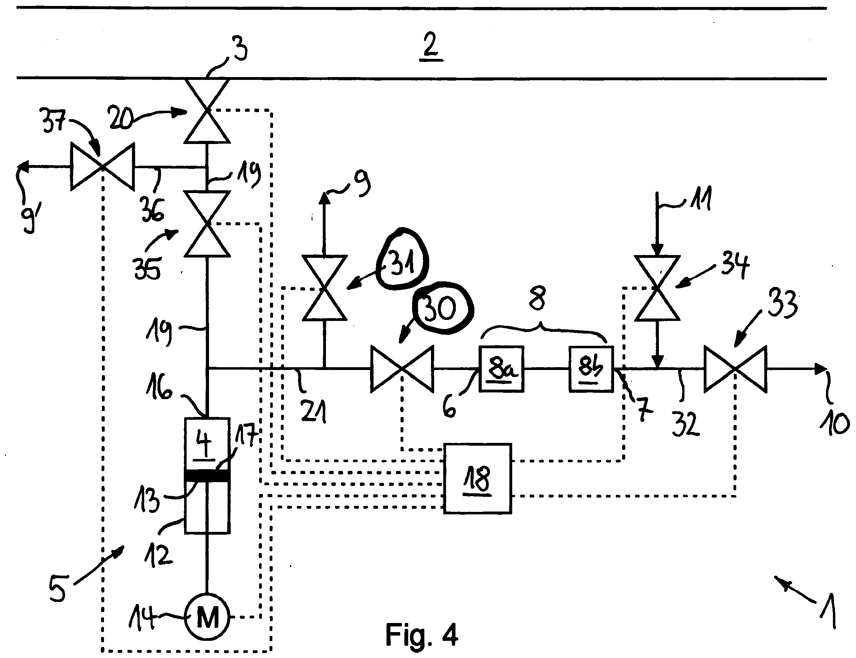

Bei dem in Fig. 4 abgebildeten vierten Ausführungsbeispiel weist die Probenentnahmevorrichtung 1 anstelle des ersten Dreiwegeventils 23 das zweite und dritte Absperrventil 30, 31 auf. Das zweite Absperrventil 30 ist zwischen der ersten Verzweigungsstelle und dem Partikelfilter 8 in der zweiten Leitung 21 angeordnet.

Die zweite Leitung 21 weist zwischen der ersten Leitung 19 und dem zweiten Absperrventil 30 eine Anschlussstelle auf, die über das dritte Absperrventil 31 mit der Auslassöffnung 9 für den Abfall verbunden ist.

Der Auslass 7 des Partikelfilters 8 ist über die dritte Leitung 32, in der das vierte Absperrventil 33 angeordnet ist, mit der Abgabeöffnung 10 für die Getränkeprobe verbunden. Zwischen dem Auslass 7 des Partikelfilters 8 und dem vierten Absperrventil 33 ist die zweite Verzweigungsstelle angeordnet, an der die dritte Leitung 32 über das fünfte Absperrventil 34 mit der Zuleitung 11 für das Rückspülmedium verbunden ist.

Bei dem vierten Ausführungsbeispiel weist der Partikelfilter 8 einen Vorfilter 8a und einen Feinfilter 8b auf, der in Strömungsrichtung der Getränkeprobe hinter dem Vorfilter 8a angeordnet ist. Der Feinfilter 8b hat einen geringeren mittleren Porendurchmesser als der Vorfilter 8a.

Im Übrigen entspricht das vierte Ausführungsbeispiel im Wesentlichen dem Ausführungsbeispiel aus Fig. 3.

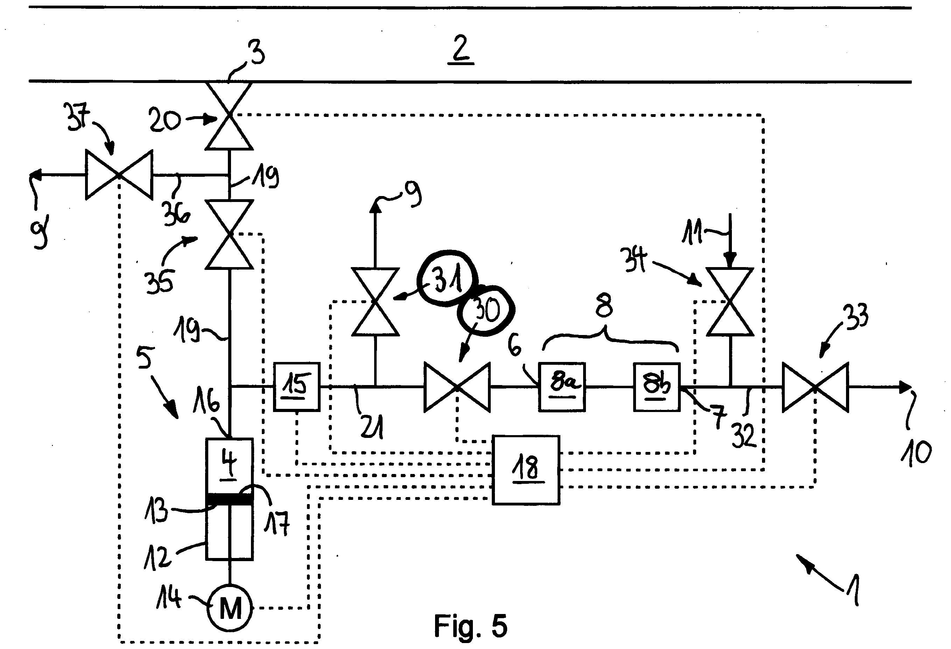

Bei dem in Fig. 5 abgebildeten fünften Ausführungsbeispiel ist in der zweiten Leitung 21 zwischen der ersten Leitung 19 und dem dritten Absperrventil ein mit der Steuereinrichtung 18 verbundener Sensor 15 zum Detektieren des Getränks angeordnet, der als pH-Wertsensor ausgestaltet ist. Das Messsignal des Sensors 15 wird in der Steuereinrichtung 18 mit einem Referenzbereich verglichen, in dem das Messsignal liegt, wenn der Sensor 15 mit der Getränkeprobe in Kontakt steht. Wenn der Sensor 15 mit dem aus der Getränkeprobe ausgetretenen Gas und/oder mit der Reinigungs-, Desinfektions- und/oder Spülflüssigkeit in Kontakt steht, weicht das Messsignal des Sensors 15 von dem Referenzbereich ab. In diesem Fall ist das zweite Absperrventil 30 in der Schließstellung verriegelt. Dadurch wird verhindert, dass Gas, Reinigungs-, Desinfektions- und/oder Spülflüssigkeit in den Partikelfilter 8 gelangt. Im Übrigen entspricht das fünfte Ausführungsbeispiel dem Ausführungsbeispiel aus Fig. 4.

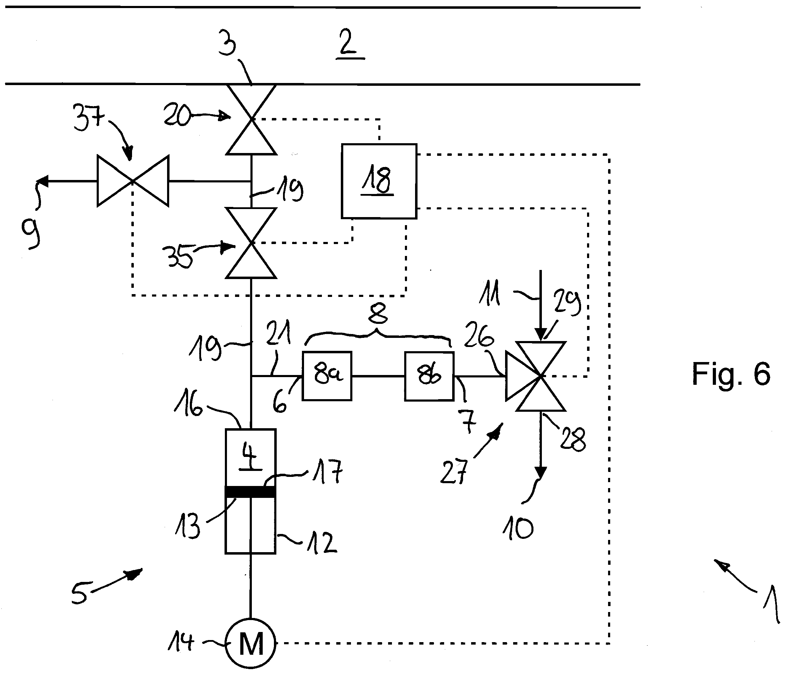

Das Leitungssystem des in Fig. 6 abgebildeten sechsten Ausführungsbeispiels entspricht dem in Fig. 3, jedoch ohne das erste Dreiwegeventil 23, d.h. die erste Leitung 19 ist in Fig. 6 direkt mit dem Einlass 6 des Partikelfilters 8 verbunden.

In der ersten Ventilkonfiguration sind zum Einbringen der Getränkeprobe in den Pumpenraum 4 des erste und sechste Absperrventil 20, 35 geöffnet und das siebte Absperrventil 37 ist geschlossen. Außerdem ist der Pumpenraum 4 durch das zweite Dreiwegeventil 27 von der Abgabeöffnung 10 für die Getränkeprobe sowie der Zuleitung 11 für das Rückspülmedium getrennt.

In der zweiten Ventilkonfiguration ist zum Entgasen der im Pumpenraum 4 befindlichen Getränkeprobe das sechste Absperrventil 35 geschlossen und der Pumpenraum 4 ist weiterhin durch das zweite Dreiwegeventil 27 von der Abgabeöffnung 10 und der Zuleitung 11 getrennt.

In der dritten Ventilkonfiguration ist das erste Absperrventil 20 geschlossen und das sechste und siebte Absperrventil 35, 37 sind geöffnet, damit das Gas aus dem Pumpenraum 4 zur der hinter dem siebten Absperrventil 37 angeordneten Auslassöffnung 9 für den Abfall abgeleitet werden kann. Über das zweite Dreiwegeventil 27 ist der Pumpenraum 4 weiterhin von der Abgabeöffnung 10 und der Zuleitung 11 getrennt.

In der vierten Ventilkonfiguration ist das erste Absperrventil 20 geschlossen und der Pumpenraum 4 ist über den Vorfilter 8a, den Feinfilter 8b und das zweite Dreiwegeventil 27 mit der Abgabeöffnung 10 verbunden.

In der fünften Ventilkonfiguration ist die Zuleitung 11 für das Rückspülmedium über das zweite Dreiwegeventil 27 mit dem Auslass 7 des Feinfilters 8b und der Einlass des Vorfilters 8a ist über das sechste und siebte Absperrventil 35, 37 mit dem Auslass 9 für den Abfall verbunden. Die Abgabeöffnung 10 ist durch zweite Dreiwegeventil 27 von der Zuleitung 11 und dem Auslass 7 des Feinfilters 8b getrennt. Das erste Absperrventil 20 ist geschlossen.

## Patentansprüche

1. Probenentnahmevorrichtung (1) zur Entnahme von Getränkeproben aus einer Getränkeleitung (2), die ein unter Druck stehendes gashaltiges Getränk enthält, wobei die Probenentnahmevorrichtung (1)
- einen Anschluss (3) für die Getränkeleitung (2),
- eine einen Antrieb (14) aufweisende Pumpe (5), die einen Pumpenraum (4) hat, der durch Wandungen (16, 17) begrenzt ist, die zum Expandieren und Komprimieren des Pumpraums (4) mittels des Antriebs (14) aufeinander zu- und voneinander wegbewegbar sind,
- eine Abgabeöffnung (10) für die Getränkeprobe,
- einen Partikelfilter (8) mit einem Einlass und einem Auslass für die Getränkeprobe, zum Filtern der Getränkeprobe,
- ein verstellbare Ventile aufweisendes Leitungssystem, welches mit dem Anschluss (3) für die Getränkeleitung (2), dem Pumpenraum (4) der Abgabeöffnung (10), dem Einlass (6) des Partikelfilters (8), dem Auslass (7) des Partikelfilters (8), einer Zuleitung (11) für ein Rückspülmedium und einer Auslassöffnung (9) für Abfall verbunden ist, und
- eine Steuereinrichtung (18) hat,
und wobei das Leitungssystem derart ausgestaltet ist und die Steuereinrichtung (18) derart mit der Pumpe (5) und den Ventilen in Steuerverbindung steht,
i) dass in einer ersten Ventilkonfiguration, bei welcher der Pumpenraum (4) mit dem Anschluss (3) für die Getränkeleitung (2) verbunden und von der Auslassöffnung (9), der Zuleitung (11) für das Rückspülmedium sowie der Abgabeöffnung (10) getrennt ist, eine Getränkeprobe, deren Volumen kleiner ist als das maximale Volumen des Pumpenraums (4), vom Anschluss (3) für die Getränkeleitung (2) in den Pumpenraum (4) einbringbar ist,
ii) dass danach eine zweite Ventilkonfiguration eingestellt wird, in welcher der Pumpenraum (4) vom Anschluss (3) für die Getränkeleitung (2), von der Auslassöffnung (9), der Zuleitung (11) für das Rückspülmedium sowie der Abgabeöffnung (10) getrennt ist, und der Pumpenraum (4) derart expandiert wird, dass zum Entgasen der Getränkeprobe ein Unterdruck im Pumpenraum entsteht,
iii) dass danach eine dritte Ventilkonfiguration eingestellt wird, in welcher der Pumpenraum (4) mit der Auslassöffnung (9) verbunden und von dem Anschluss (3) für die Getränkeleitung (2), der Zuleitung (11) für das Rückspülmedium sowie der Abgabeöffnung (10) getrennt ist, und der Pumpenraum (4) derart komprimiert wird, dass ein in einem oberen Teil des Pumpenraums (4) befindliches erstes Teilvolumen zu der Auslassöffnung (9) hin verdrängt und ein in einem unteren Teil des Pumpenraums (4) befindliches zweites Teilvolumen im Pumpenraum (4) verbleibt,
iv) dass danach eine vierte Ventilkonfiguration eingestellt wird, in welcher der Pumpenraum (4) über den Partikelfilter (8) mit der Abgabeöffnung (10) verbunden und von dem Anschluss (3) für die Getränkeleitung (2), der Zuleitung (11) für das Rückspülmedium sowie der Auslassöffnung (9) getrennt ist, und der Pumpenraum (4) zum Verdrängen der darin befindlichen Getränkeprobe zur Abgabeöffnung (10) hin weiter komprimiert wird, und
(v) dass danach zum Rückspülen des Partikelfilters (8) eine fünfte Ventilkonfiguration eingestellt wird, bei der die Zuleitung (11) für das Rückspülmedium über den Partikelfilter (8) mit der Auslassöffnung (9) verbunden ist.

2. Probenentnahmevorrichtung (1) nach Anspruch 1, **dadurch gekennzeichnet, dass** die Steuereinrichtung (18) derart ausgestaltet ist, dass die Schritte ii) und iii) aus Anspruch 1 mindestens einmal wiederholt werden, bevor Schritt iv) aus Anspruch 1 durchgeführt wird.

3. Probenentnahmevorrichtung (1) nach Anspruch 1 oder 2, **dadurch gekennzeichnet, dass** in der zweiten und/oder dritten Ventilkonfiguration der Pumpenraum (4) vom Einlass (6) des Partikelfilters (8) getrennt ist.

4. Probenentnahmevorrichtung (1) nach einem der Ansprüche 1 bis 3,
**dadurch gekennzeichnet, dass** der Partikelfilter (8) einen Vorfilter (8a) und einen damit in Reihe geschalteten Feinfilter (8b) aufweist, und dass der Einlass (6) des Partikelfilters (8) am Vorfilter (8a) und der Auslass (7) des Partikelfilters (8) am Feinfilter (8b) angeordnet ist.

5. Probenentnahmevorrichtung (1) nach einem der Ansprüche 1 bis 4,
**dadurch gekennzeichnet, dass** der Anschluss (3) für die Getränkeleitung (2) über eine erste Leitung (19) mit dem Pumpenraum (4) verbunden ist, dass in der ersten Leitung (19) ein erstes Absperrventil (20) angeordnet ist, dass der Pumpenraum (4) über eine zweite Leitung (21), die in den Pumpenraum (4) mündet oder an einer zwischen diesem und dem ersten Absperrventil (20) angeordneten ersten Verzweigungsstelle an der ersten Leitung (19) angeschlossen ist, mit einem ersten Fluidanschluss (22) eines ersten Dreiwegeventils (23) verbunden ist, dass ein zweiter Fluidanschluss (24) des ersten Dreiwegeventils (23) mit der Auslassöffnung (10) und ein dritter Fluidanschluss (25) des ersten Dreiwegeventils (23) mit dem Einlass (6) des Partikelfilters (8) verbunden ist.

6. Probenentnahmevorrichtung (1) nach einem der Ansprüche 1 bis 4,
**dadurch gekennzeichnet, dass** der Anschluss (3) für die Getränkeleitung (2) über eine erste Leitung (19) mit dem Pumpenraum (4) verbunden ist, dass in der ersten Leitung (19) ein erstes Absperrventil (20) angeordnet ist, dass der Pumpenraum (4) über eine zweite Leitung (21), die in den Pumpenraum (4) mündet oder an einer zwischen diesem und dem ersten Absperrventil (20) angeordneten ersten Verzweigungsstelle an der ersten Leitung (19) angeschlossen ist, mit dem Einlass (6) des Partikelfilters (8) verbunden ist, dass in der zweiten Leitung (21) ein zweites Absperrventil (30) angeordnet ist, und am Pumpenraum (4) und/oder an der ersten Leitung (19) zwischen dem Pumpenraum (4) und dem ersten Absperrventil (20) und/oder an der zweiten Leitung (21) zwischen dem Pumpenraum (4) und dem zweiten Absperrventil (30) eine Anschlussstelle vorgesehen ist, die über ein drittes Absperrventil (31) mit der Auslassöffnung (9) für den Abfall verbunden ist.

7. Probenentnahmevorrichtung (1) nach Anspruch 5 oder 6, **dadurch gekennzeichnet, dass** in dem Leitungssystem zwischen dem Anschluss (3) für die Getränkeleitung (2) und dem Einlass (6) des Partikelfilters (8) und/oder zwischen dem Pumpenraum (4) und dem Einlass (6) des Partikelfilters (8) mindestens ein mit der Steuereinrichtung (18) verbundener Sensor (15) zum Detektieren des Getränks angeordnet ist, und dass die Steuereinrichtung (18) derart ausgestaltet ist, dass der Einlass (6) des Partikelfilters (8) mittels des ersten Dreiwegeventils (23) oder des zweiten Absperrventils (30) abgesperrt ist, wenn der Sensor (15) das Getränk nicht detektiert.

8. Probenentnahmevorrichtung (1) nach Anspruch 7, **dadurch gekennzeichnet, dass** der Sensor (15) einen Leitfähigkeitssensor und/oder einen pH-Wertsensor aufweist.

9. Probenentnahmevorrichtung (1) nach einem der Ansprüche 1 bis 8,
**dadurch gekennzeichnet, dass** der Auslass (7) des Partikelfilters (8) mit einem ersten Fluidanschluss (26) eines zweiten Dreiwegeventils (27) verbunden ist, dass ein zweiter Fluidanschluss (28) des zweiten Dreiwegeventils (27) mit der Abgabeöffnung (9) für die Getränkeprobe und ein dritter Fluidanschluss (29) des zweiten Dreiwegeventils (27) mit der Zuleitung (11) für das Rückspülmedium verbunden ist.

10. Probenentnahmevorrichtung (1) nach einem der Ansprüche 1 bis 8,
**dadurch gekennzeichnet, dass** der Auslass (7) des Partikelfilters über eine dritte Leitung (32), in der ein viertes Absperrventil (33) angeordnet ist, mit der Abgabeöffnung (10) für die Getränkeprobe verbunden ist, und dass zwischen dem Auslass (7) des Partikelfilters (8) und dem vierten Absperrventil (33) eine zweite Verzweigungsstelle angeordnet ist, an der die dritte Leitung (32) über ein fünftes Absperrventil (34) mit der Zuleitung (11) für das Rückspülmedium verbunden ist.

11. Probenentnahmevorrichtung (1) nach einem der Ansprüche 1 bis 10, **dadurch gekennzeichnet, dass** der Anschluss (3) für die Getränkeleitung (2) über eine erste Leitung (19) mit dem Pumpenraum (4) verbunden ist, dass in der ersten Leitung (19) das erste Absperrventil (20) und ein sechstes Absperrventil (35) in Reihe geschaltet sind, dass der Pumpenraum (4) oder eine zwischen dem sechsten Absperrventil (35) und dem Pumpenraum (4) in der ersten Leitung (19) vorgesehene erste Verzweigungsstelle über die zweite Leitung (21) mit dem Einlass (6) des Partikelfilters (8) verbunden ist, und dass zwischen dem ersten Absperrventil (20) und dem sechsten Absperrventil (35) eine dritte Verzweigungsstelle angeordnet ist, an der die erste Leitung (19) über eine vierte Leitung (36), in der ein siebtes Absperrventil (37) angeordnet ist, mit der Auslassöffnung (9) oder einer weiteren Auslassöffnung (9') für Abfall verbunden ist.

## Claims

1. Sampling device (1) for taking beverage samples from a beverage line (2) containing a pressurized carbonated beverage, wherein the sampling device (1) has
- a connection (3) for the beverage line (2),
- a pump (5) having a drive (14), which pump has a pump chamber (4) that is delimited by walls (16, 17) that can be moved towards and away from one another, in order to expand and compress the pump chamber (4) by means of the drive (14),
- a discharge opening (10) for the beverage sample,
- a particulate filter (8) with an inlet and an outlet for the beverage sample, for filtering the beverage sample,
- a line system with adjustable valves, which is connected to the connection (3) for the beverage line (2), the pump chamber (4), the discharge opening (10), the inlet (6) of the particulate filter (8), the outlet (7) of the particulate filter (8), a supply line (11) for a backflushing medium and a waste outlet opening (9), and
- a control device (18),
and wherein the line system is configured in such a manner and the control device (18) is in control connection with the pump (5) and the valves in such a manner
i) that in a first valve configuration, in which the pump chamber (4) is connected to the connection (3) for the beverage line (2) and is separated from the outlet opening (9), the supply line (11) for the backflushing medium and the discharge opening (10), a beverage sample, the volume of which is smaller than the maximum volume of the pump chamber (4), can be introduced into the pump chamber (4) from the connection (3) for the beverage line (2),
ii) that thereafter a second valve configuration is set, in which the pump chamber (4) is separated from the connection (3) for the beverage line (2), from the outlet opening (9), the supply line (11) for the backflushing medium and the discharge opening (10), and the pump chamber (4) is expanded in such a manner that a vacuum is created in the pump chamber, in order to degas the beverage sample,
iii) that thereafter a third valve configuration is set, in which the pump chamber (4) is connected to the outlet opening (9) and is separated from the connection (3) for the beverage line (2), the supply line (11) for the backflushing medium and the discharge opening (10), and the pump chamber (4) is compressed in such a manner that a first partial volume located in an upper part of the pump chamber (4) is displaced towards the outlet opening (9) and a second partial volume located in a lower part of the pump chamber (4) remains in the pump chamber (4),
iv) that thereafter a fourth valve configuration is set, in which the pump chamber (4) is connected to the discharge opening (10) via the particulate filter (8) and is separated from the connection (3) for the beverage line (2), the supply line (11) for the backflushing medium and the outlet opening (9), and the pump chamber (4) is further compressed to displace the sample located therein towards the discharge opening (10).
v) that thereafter a fifth valve configuration is set, in order to backflush the particulate filter (8), in which configuration the supply line (11) for the backflushing medium is connected to the outlet opening (9) via the particulate filter (8).

2. Sampling device (1) according to Claim 1, **characterized in that** the control device (18) is configured in such a way that steps ii) and iii) from Claim 1 are repeated at least once before step iv) from Claim 1 is executed.

3. Sampling device (1) according to Claim 1 or 2, **characterized in that** in the second and/or third valve configuration, the pump chamber (4) is separated from the inlet (6) of the particulate filter (8).

4. Sampling device (1) according to one of Claims 1 to 3, **characterized in that** the particulate filter (8) has a pre-filter (8a) and a fine filter (8b) connected in series thereto, and that the inlet (6) of the particulate filter (8) is arranged at the pre-filter (8a) and the outlet (7) of the particulate filter (8) is arranged at the fine filter (8b).

5. Sampling device (1) according to one of Claims 1 to 4, **characterized in that** the connection (3) for the beverage line (2) is connected to the pump chamber (4) via a first line (19), that a first shut-off valve (20) is arranged in the first line (19), that the pump chamber (4) is connected to a first fluid port (22) of a first three-way valve (23) via a second line (21) which opens into the pump chamber (4) or is connected to the first line (19) at a first branching point arranged between said pump chamber and the first shut-off valve (20), that a second fluid port (24) of the first three-way valve (23) is connected to the outlet opening (10) and a third fluid port (25) of the first three-way valve (23) is connected to the inlet (6) of the particulate filter (8).

6. Sampling device (1) according to one of Claims 1 to 4, **characterized in that** the connection (3) for the beverage line (2) is connected to the pump chamber (4) via a first line (19), that a first shut-off valve (20) is arranged in the first line (19), that the pump chamber (4) is connected to the inlet (6) of the particulate filter (8) via a second line (21) which opens into the pump chamber (4) or is connected to the first line (19) at a first branching point arranged between said pump chamber and the first shut-off valve (20), that a second shut-off valve (30) is arranged in the second line (21) and at the pump chamber (4) and/or at the first line (19) between the pump chamber (4) and the first shut-off valve (20) and/or at the second line (21) between the pump chamber (4) and the second shut-off valve (30), a connection point is provided that is connected to the waste outlet opening (9) via a third shut-off valve (31).

7. Sampling device (1) according to Claim 5 or 6, **characterized in that** at least one sensor (15) connected to the control device (18) for detecting the beverage is arranged in the line system between the connection (3) for the beverage line (2) and the inlet (6) of the particulate filter (8) and/or between the pump chamber (4) and the inlet (6) of the particulate filter (8), and that the control device (18) is configured in such a manner that the inlet (6) of the particulate filter (8) is blocked by means of the first three-way valve (23) or the second shut-off valve (30) when the sensor (15) does not detect the beverage.

8. Sampling device (1) according to Claim 7, **characterized in that** the sensor (15) comprises a conductivity sensor and/or a pH sensor.

9. Sampling device (1) according to one of Claims 1 to 8, **characterized in that** the outlet (7) of the particulate filter (8) is connected to a first fluid port (26) of a second three-way valve (27), that a second fluid port (28) of the second three-way valve (27) is connected to the discharge opening (9) for the beverage sample and a third fluid port (29) of the second three-way valve (27) is connected to the supply line (11) for the backflushing medium.

10. Sampling device (1) according to one of Claims 1 to 8, **characterized in that** the outlet (7) of the particulate filter is connected to the discharge opening (10) for the beverage sample via a third line (32), in which a fourth shut-off valve (33) is arranged, and that between the outlet (7) of the particulate filter (8) and the fourth shut-off valve (33) a second branching point is arranged, at which the third line (32) is connected via a fifth shut-off valve (34) to the supply line (11) for the backflushing medium.

11. Sampling device (1) according to one of Claims 1 to 10, **characterized in that** the connection (3) for the beverage line (2) is connected to the pump chamber (4) via a first line (10), that the first shut-off valve (20) and a sixth shut-off valve (35) are connected in series in the first line (19), that the pump chamber (4), or a first branching point provided in the first line (19) between the sixth shut-off valve (35) and the pump chamber (4), is connected to the inlet (6) of the particulate filter (8) via the second line (21), and that a third branching point is arranged between the first shut-off valve (20) and the sixth shut-off valve (35), at which the first line (19) is connected to the outlet opening (9), or to a further waste outlet opening (9), via a fourth line (36) in which a seventh shut-off valve (37) is arranged.

## Revendications

1. Dispositif d'échantillonnage (1) permettant le prélèvement d'échantillons de boisson d'une conduite de boisson (2), laquelle contient une boisson gazeuse pressurisée, le dispositif d'échantillonnage (1) ayant
- un raccordement (3) pour la conduite de boisson (2),
- une pompe (5) présentant un entraînement (14), laquelle pompe a une chambre de pompe (4) qui est délimitée par des parois (16, 17) qui peuvent être rapprochées et éloignées l'une de l'autre au moyen de l'entraînement (14) pour l'expansion et la compression de la chambre de pompe (4),
- une ouverture de distribution (10) pour l'échantillon de boisson,
- un filtre à particules (8) doté d'une entrée et d'une sortie pour l'échantillon de boisson, pour la filtration de l'échantillon de boisson,
- un système de conduites présentant des soupapes réglables, lequel est relié au raccordement (3) pour la conduite de boisson (2), à la chambre de pompe (4), à l'ouverture de distribution (10), à l'entrée (6) du filtre à particules (8), à la sortie (7) du filtre à particules (8), à une conduite d'amenée (11) pour un milieu de lavage à contrecourant et à une ouverture de sortie (9) pour des déchets, et
- un dispositif de commande (18),
et le système de conduites étant configuré de telle sorte et le dispositif de commande (18) étant en liaison de commande avec la pompe (5) et les soupapes de telle sorte
i) que dans une première configuration de soupapes, dans laquelle la chambre de pompe (4) est reliée au raccordement (3) pour la conduite de boisson (2) et est séparée de l'ouverture de sortie (9), de la conduite d'amenée (11) pour le milieu de lavage à contrecourant ainsi que de l'ouverture de distribution (10), un échantillon de boisson, dont le volume est inférieur au volume maximal de la chambre de pompe (4), peut être introduit à partir du raccordement (3) pour la conduite de boisson (2) dans la chambre de pompe (4),
ii) qu'ensuite une deuxième configuration de soupapes est réglée, dans laquelle la chambre de pompe (4) est séparée du raccordement (3) pour la conduite de boisson (2), de l'ouverture de sortie (9), de la conduite d'amenée (11) pour le milieu de lavage à contrecourant ainsi que de l'ouverture de distribution (10), et la chambre de pompe (4) est agrandie de telle sorte qu'une dépression soit produite dans la chambre de pompe pour le dégazage de l'échantillon de boisson,
iii) qu'ensuite une troisième configuration de soupapes est réglée, dans laquelle la chambre de pompe (4) est reliée à l'ouverture de sortie (9) et séparée du raccordement (3) pour la conduite de boisson (2), de la conduite d'amenée (11) pour le milieu de lavage à contrecourant ainsi que de l'ouverture de distribution (10), et la chambre de pompe (4) est comprimée de telle sorte qu'un premier volume partiel se trouvant dans une partie supérieure de la chambre de pompe (4) soit refoulé vers l'ouverture de sortie (9) et qu'un deuxième volume partiel se trouvant dans une partie inférieure de la chambre de pompe (4) demeure dans la chambre de pompe (4),
iv) qu'ensuite une quatrième configuration de soupapes est réglée, dans laquelle la chambre de pompe (4) est reliée à l'ouverture de distribution (10) par le biais du filtre à particules (8) et séparée du raccordement (3) pour la conduite de boisson (2), de la conduite d'amenée (11) pour le milieu de lavage à contrecourant ainsi que de l'ouverture de sortie (9), et la chambre de pompe (4) est comprimée davantage pour refouler l'échantillon de boisson qui s'y trouve vers l'ouverture de distribution (10), et
(v) qu'ensuite pour le lavage à contrecourant du filtre à particules (8) une cinquième configuration de soupapes est réglée, dans laquelle la conduite d'amenée (11) pour le milieu de lavage à contrecourant est reliée à l'ouverture de sortie (9) par le biais du filtre à particules (8).

2. Dispositif d'échantillonnage (1) selon la revendication 1, **caractérisé en ce que** le dispositif de commande (18) est configuré de telle sorte que les étapes ii) et iii) de la revendication 1 sont répétées au moins une fois, avant que l'étape iv) de la revendication 1 ne soit effectuée.

3. Dispositif d'échantillonnage (1) selon la revendication 1 ou 2, **caractérisé en ce que**, dans la deuxième et/ou la troisième configuration de soupapes, la chambre de pompe (4) est séparée de l'entrée (6) du filtre à particules (8).

4. Dispositif d'échantillonnage (1) selon l'une des revendications 1 à 3, **caractérisé en ce que** le filtre à particules (8) présente un préfiltre (8a) et un filtre fin (8b) monté en série avec celui-ci, et **en ce que** l'entrée (6) du filtre à particules (8) est disposée au niveau du préfiltre (8a) et la sortie (7) du filtre à particules (8) est disposée au niveau du filtre fin (8b).

5. Dispositif d'échantillonnage (1) selon l'une des revendications 1 à 4, **caractérisé en ce que** le raccordement (3) pour la conduite de boisson (2) est relié à la chambre de pompe (4) par le biais d'une première conduite (19), **en ce qu'**une première soupape d'arrêt (20) est disposée dans la première conduite (19), **en ce que** la chambre de pompe (4) est reliée à un premier raccordement de fluide (22) d'une première soupape à trois voies (23) par le biais d'une deuxième conduite (21) qui débouche dans la chambre de pompe (4) ou est raccordée à la première conduite (19) au niveau d'un premier point de bifurcation disposé entre cette chambre de pompe et la première soupape d'arrêt (20), **en ce qu'**un deuxième raccordement de fluide (24) de la première soupape à trois voies (23) est relié à l'ouverture de sortie (10) et un troisième raccordement de fluide (25) de la première soupape à trois voies (23) est relié à l'entrée (6) du filtre à particules (8).

6. Dispositif d'échantillonnage (1) selon l'une des revendications 1 à 4, **caractérisé en ce que** le raccordement (3) pour la conduite de boisson (2) est relié à la chambre de pompe (4) par le biais d'une première conduite (19), **en ce qu'**une première soupape d'arrêt (20) est disposée dans la première conduite (19), **en ce que** la chambre de pompe (4) est reliée à l'entrée (6) du filtre à particules (8) par le biais d'une deuxième conduite (21) qui débouche dans la chambre de pompe (4) ou est raccordée à la première conduite (19) au niveau d'un premier point de bifurcation disposé entre cette chambre de pompe et la première soupape d'arrêt (20), **en ce qu'**une deuxième soupape d'arrêt (30) est disposée dans la deuxième conduite (21), et un point de raccordement est prévu au niveau de la chambre de pompe (4) et/ou au niveau de la première conduite (19) entre la chambre de pompe (4) et la première soupape d'arrêt (20) et/ou au niveau de la deuxième conduite (21) entre la chambre de pompe (4) et la deuxième soupape d'arrêt (30), lequel point de raccordement est relié à l'ouverture de sortie (9) pour les déchets par le biais d'une troisième soupape d'arrêt (31).

7. Dispositif d'échantillonnage (1) selon la revendication 5 ou 6, **caractérisé en ce qu'**au moins un capteur (15) pour la détection de la boisson relié au dispositif de commande (18) est disposé dans le système de conduites entre le raccordement (3) pour la conduite de boisson (2) et l'entrée (6) du filtre à particules (8) et/ou entre la chambre de pompe (4) et l'entrée (6) du filtre à particules (8), et **en ce que** le dispositif de commande (18) est configuré de telle sorte que l'entrée (6) du filtre à particules (8) est bloquée au moyen de la première soupape à trois voies (23) ou de la deuxième soupape d'arrêt (30) lorsque le capteur (15) ne détecte pas la boisson.

8. Dispositif d'échantillonnage (1) selon la revendication 7, **caractérisé en ce que** le capteur (15) présente un capteur de conductivité et/ou un capteur de valeur pH.

9. Dispositif d'échantillonnage (1) selon l'une des revendications 1 à 8, **caractérisé en ce que** la sortie (7) du filtre à particules (8) est reliée à un premier raccordement de fluide (26) d'une deuxième soupape à trois voies (27), **en ce qu'**un deuxième raccordement de fluide (28) de la deuxième soupape à trois voies (27) est relié à l'ouverture de distribution (9) pour l'échantillon de boisson et un troisième raccordement de fluide (29) de la deuxième soupape à trois voies (27) est relié à la conduite d'amenée (11) pour le milieu de lavage à contrecourant.

10. Dispositif d'échantillonnage (1) selon l'une des revendications 1 à 8, **caractérisé en ce que** la sortie (7) du filtre à particules est reliée à l'ouverture de distribution (10) pour l'échantillon de boisson par le biais d'une troisième conduite (32) dans laquelle une quatrième soupape d'arrêt (33) est disposée, et **en ce qu'**un deuxième point de bifurcation est disposé entre la sortie (7) du filtre à particules (8) et la quatrième soupape d'arrêt (33), point de bifurcation au niveau duquel la troisième conduite (32) est reliée à la conduite d'amenée (11) pour le milieu de lavage à contrecourant par le biais d'une cinquième soupape d'arrêt (34).

11. Dispositif d'échantillonnage (1) selon l'une des revendications 1 à 10, **caractérisé en ce que** le raccordement (3) pour la conduite de boisson (2) est relié à la chambre de pompe (4) par le biais d'une première conduite (19), **en ce que**, dans la première conduite (19), la première soupape d'arrêt (20) et une sixième soupape d'arrêt (35) sont montées en série, **en ce que** la chambre de pompe (4) ou un premier point de bifurcation prévu entre la sixième soupape d'arrêt (35) et la chambre de pompe (4) dans la première conduite (19) est relié à l'entrée (6) du filtre à particules (8) par le biais de la deuxième conduite (21), et **en ce qu'**un troisième point de bifurcation est disposé entre la première soupape d'arrêt (20) et la sixième soupape d'arrêt (35), point de bifurcation au niveau duquel la première conduite (19) est reliée à l'ouverture de sortie (9) ou à une autre ouverture de sortie (9') pour les déchets par le biais d'une quatrième conduite (36) dans laquelle une septième soupape d'arrêt (37) est disposée.
